# EUROPEAN PATENT APPLICATION

(11) **EP 2 279 714 A1**
(43) Date of publication of application: **02.02.2011**
(21) Application number: 09166804.6
(22) Date of filing: 30.07.2009
(51) Int. Cl.: A61F 13/15, A61F 13/56, A61F 13/60, A61F 13/62

(54) **Absorbent articles with adhesive portion and protrusions**

(71) Applicant: The Procter and Gamble Company, Cincinnati, OH 45202 (US)
(72) Inventor: Bonelli, Guido, 65129 PESCARA (IT); Bellucci, Remo, 65010, SPOLTORE (PESCARA) (IT)
(74) Representative: Briatore, Andrea

(57) **Abstract**

Absorbent articles for personal hygiene comprising an adhesive portion, protrusions, separate from the adhesive portion and folding lines configured so that the article can be folded along the folding lines so that the adhesive portion comes in contact with the protrusions. The articles of the present invention can be packaged in a folded configuration and require less or no release paper or film to protect the adhesive on their garment facing surface.

## Description

### FIELD OF THE INVENTION

The present invention relates to absorbent articles for personal hygiene of the type which are positioned between the undergarment and the body of the user when in use, for example feminine hygiene articles such as sanitary napkins, pantyliners, incontinence pads and the like. The articles of the invention have an improved garment fastening system.

### BACKGROUND OF THE INVENTION

Absorbent articles for personal hygiene, in particular sanitary napkins, pantiliners or incontinence pads, are commonly commercialized in carton boxes or soft bags comprising several articles. The articles are sometimes individually wrapped in a thin nonwoven paper or plastic film. In order to reduce the dimensions of the packaging and make the articles more practical to carry around in a purse, the absorbent articles are commonly packaged in a folded configuration. The articles typically comprise from 1 or 2 folding lines, which are parallel to each other and oriented in transverse direction (i.e. in a direction perpendicular to the longitudinal axis of the article). If wings are present they are normally folded over the topsheet of the articles along a longitudinal folding line.

The garment facing surface of these absorbent articles typically comprise a garment fastening system which, in the majority of the currently marketed absorbent articles, comprise a continuous or discontinuous layer of pressure sensitive adhesive which extends over at least a significant portion of the garment facing surface of the article. When these articles are packaged, a siliconized release paper or film is generally used to entirely cover the adhesive in order to protect it before use from unwanted adhesions or contaminations. When the absorbent articles comprise wings, these normally also include an adhesive patch and an additional layer of release paper or film is used in order to protect this adhesive.

The release paper or film is removed by the user before using the product in order to release the adhesive so that the article can adhere to the undergarment. The release paper or film is normally discarded after removal and therefore it represents an annoyance, an environmental concern and moreover, being a silicone treated material, it is a relatively expensive element which does not improve the performance of the absorbent articles when in use.

Over the years there have been many attempts to improve the adhesive release systems, for example the Procter & Gamble Company currently markets under the trade name Always™ folded absorbent articles where the release film is slightly larger than the article itself and also acts as a wrapper film for the article. This solution, while allowing a single material to perform two functions, still requires the use of a siliconized film for the wrapper film material which is more expensive than the simple untreated plastic, paper or nonwoven film which could be used for wrapping the same article if the adhesive release function was not required.

Mechanical fastening systems such as hooks or stems have also been proposed. Mechanical fastening systems are not adhesive per se and therefore absorbent articles comprising only mechanical fastening systems do not require a release paper or film. However, a purely mechanical fastening system is not able in some cases to ensure perfect adhesion on all undergarment materials. Moreover the strongest mechanical fastening systems such as those having hooks (like Velcro™) may cause damage to delicate undergarments. More delicate mechanical fastening systems such as those based on stems, like those described in European Patent application EP2014270, are desirably used in combination with an pressure sensitive adhesive in order to ensure optimal adhesion stability in response to forces acting in all directions and therefore still require a release paper or film.

Accordingly it is still desirable to develop new absorbent articles which offer optimal adhesion and garment safety and which can be packaged in a folded configuration requiring less or no release paper or film to protect the adhesive on their garment facing surface.

### SUMMARY OF THE INVENTION

The present invention relates to absorbent articles for personal hygiene of the type which are positioned between the undergarment and the body of the users when in use, for example feminine hygiene articles such as sanitary napkins, pantyliners, incontinence pads and the like. The articles according to the invention comprise a body facing surface and a garment facing surface. The garment facing surface of the articles comprises an adhesive portion and protrusions separate from the adhesive portion. The articles of the present invention also comprise one or more folding lines. The adhesive portion and the protrusions are configured so that the article can be folded along the folding lines so that at least a part of the adhesive portion comes in contact with the protrusions.

The articles of the present invention can be packaged in a folded configuration and require less or no release paper or film for protecting the adhesive on their garment facing surface.

### BRIEF DESCRIPTION OF THE DRAWINGS

Figure 1 shows the garment facing side of an embodiment of the invention in the form of an absorbent pad 10 comprising one folding line 20, an adhesive portion formed by two discrete areas 30 and a multiplicity of protrusions 40.
Fig. 1A is a side view of the embodiment of Fig. 1, showing in broken lines the position of the pad when partially folded over its backsheet along the folding line 20 in a V shaped configuration.
Fig. 1B is a side view of the embodiment of Fig. 1 completely folded over the backsheet in a V shaped configuration, with the adhesive portion 30 and the protrusions 40 positioned so that the adhesive portion comes in contact with the protrusions.
Fig. 2 shows another embodiment of the invention having three folding lines 20 and the adhesive portions being formed by four discrete areas.
Fig. 2A is a side view of the embodiment of Fig. 2, showing in broken lines the position of the pad when partially folded over its backsheet along the folding lines 20 in a W shaped configuration.
Fig. 2B is a side view of the embodiment of Fig. 1 completely folded over the backsheet in a W shaped configuration.
Fig. 3 shows the garment facing side of another embodiment of the invention similar to the embodiment of Fig. 1, but wherein the adhesive portion and the protrusions are obtained by attaching a plastic strip 60 on the backsheet of the article, the strip including adhesive areas 30 and areas with protrusions 40.
Fig. 4 shows the garment facing side of another embodiment according to the invention with three folding lines and wherein the adhesive portions and the protrusions are obtained by attaching trapezoidal pieces from a plastic strip, each piece comprising an adhesive area 30 and two areas with protrusions 40.
Fig. 5 shows the garment facing side of another embodiment of the invention, wherein the folding lines 20 are directed in longitudinal direction and the adhesive portion if formed by two adhesive areas having a semicircular shape.
Fig. 6 shows a cross-sectional view of one embodiment of the protrusions 40 according to the invention wherein the protrusions are stems projecting out of a base strip 1 and have a lower section of low conicity 2.

### DETAILED DESCRIPTION OF THE INVENTION

By "absorbent articles for personal hygiene which are positioned between the undergarment and the body of the user when in use" we mean absorbent articles worn externally to collect bodily fluid such as menses, urine or feces or blood in particular feminine hygiene articles like sanitary napkins, pantiliners, incontinence pads and the like. These articles typically comprise a fluid pervious topsheet, a backsheet that may be fluid impervious and an absorbent element positioned therebetween.

By "backsheet" it is meant the layer of the article which is lowermost toward the garment facing surface of the article and on which the adhesive portion and protrusions are positioned. Advantageously, the backsheet may be substantially liquid impervious.

By "garment facing surface of the article", we mean the surface of the article which faces the inside of the undergarment of the user when in use. The "body facing surface" is the surface of the article opposed to the "garment facing surface". The garment facing surface of the article does not always coincide with the garment facing side of the backsheet because if, for example, an adhesive is applied onto a portion of the garment facing side of the backsheet, the adhesive, being the outermost material will be part of the garment facing surface of the article, while the portion of backsheet covered by the adhesive will not be part of the garment facing surface of the article. According to this definition, in the case of an absorbent article having wings extending outside the undergarment when in use, the wings are not considered part of the "garment facing surface".

By "pressure sensitive adhesive" it is meant an adhesive that can be attached to a user's undergarment with the pressure of a finger.

By "adhesive portion" it is meant the portion of the garment facing surface of the article which has an adhesive applied onto it, typically a pressure sensitive adhesive. The adhesive can be applied on the garment facing surface of the article by any standard method. For example it can be coated onto a portion of the backsheet or a strip of adhesive tape can be attached onto the backsheet so that its adhesive face constitutes part of the garment facing surface of the article.

By "protrusions" it is meant material protruding outside the plane defined by the backsheet. Protrusions can be formed directly onto the backsheet by mechanical deformation of the backsheet itself or can be obtained by attaching onto the backsheet elements which can perform this function either as individual elements or in the form of a base strip from which the elements protrude. Protrusions typically have an elongated shape so that their width, corresponding to their largest dimension measured parallel to a plane defined by the backsheet of the article, and their height, measured along a direction perpendicular to the plane are such that the ratio of height/width is higher than 0.5 or higher than 1.0 or higher than 1.5. In some cases their width can be of from about 0.01mm to about 5mm, or from about 0.05mm to about 1mm, or from about 0.05 to about 0.250mm and their height can be from about 0.005mm to about 5mm, or from about 0.025mm to about 4mm or from about 0.5 to about 3mm.

By "bodily fluid" it is meant any fluid produced by the human body including for instance perspiration, urine, blood, menstrual fluids, vaginal secretions and the like.

The term "disposable" is used herein to describe articles, which are not intended to be laundered or otherwise restored or reused as an article (i.e. they are intended to be discarded after a single use and conventionally to be recycled, composted or otherwise disposed of in an environmentally compatible manner). The articles of the inventions may be disposable.

The term "in use", as used herein refers to the period of time that starts when the absorbent article is placed in the undergarment of the wearer until removal of the absorbent article for disposal.

The term "stem" as used herein refers to an elongated protrusion having a substantially straight longitudinal axis and a cross section area, on a plane perpendicular to the longitudinal axis, that is constant or decreasing from the base to the top of the stem. Stems can penetrate a fabric among the threads, without grasping them, as opposed to a "hook" type protrusions, like those used in Velcro ®, which are protrusions terminating with a hook which cannot penetrate a fabric but grasp the threads of the fabric.

The term "conical" as used herein refers to a shape which cross sectional area measured on a plane perpendicular to the axis of the shape, decreases towards its top portion.

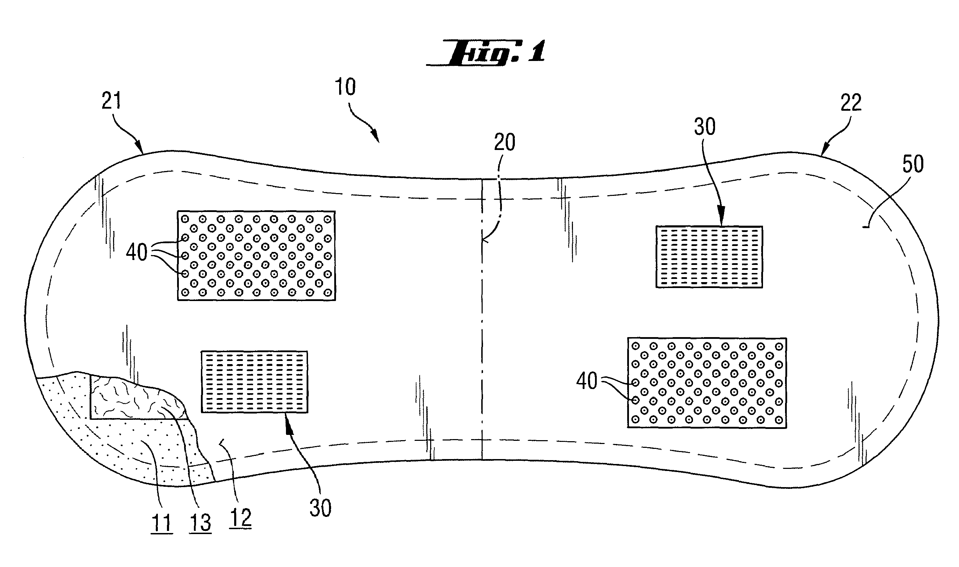

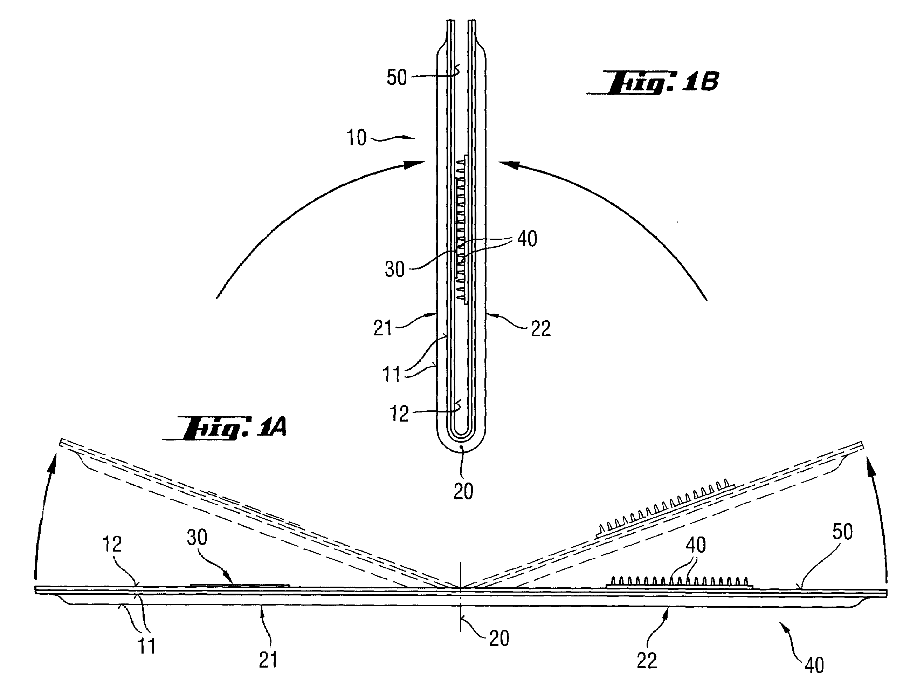

The present invention can be better understood by reference to the attached drawings which show exemplary embodiments of the invention. Turning now to Fig. 1, an absorbent article 10 according to the invention may comprise a fluid pervious topsheet 11, a backsheet 12 which may be liquid impervious and an absorbent element (or absorbent "core") 13 positioned therebetween. Other layers such as a secondary topsheet or a wrap for the absorbent element can also be present.

The topsheet 11 is the layer of the article which is oriented towards and contacts the body of the wearer, and is therefore the first layer to receive the bodily discharges. The topsheet is normally made of a single layer, as represented in the Figures, but may also comprises more than one layer (for example a central topsheet layer and two overlapping lateral stripes, as disclosed in WO93/09744 or EP766,953).

The topsheet 11 is normally liquid pervious. The term "liquid pervious" as used herein refers to components that allow liquids to pass therethrough without significantly retarding or obstructing the transmission of such liquids therethrough.

It is envisaged that any conventional topsheet material may be used within the invention. Suitable topsheets may be made from nonwoven materials or perforated polyolefinic films. An exemplary topsheet suitable for use herein is a relatively hydrophobic 20 gsm spunbonded nonwoven web comprising bicomponent fibers of the sheath core type (PP/PE) available from Pegas a.s., of the Czech Republic.

If desired, the topsheet 11 may be sprayed with a surfactant to enhance liquid penetration to the core. The surfactant is typically non-ionic and should be nonirritating to the skin. A surfactant density of about 0.01 milligrams per square centimeter of topsheet area is normally suitable. An exemplary surfactant is sold by the Glyco Chemical, Inc. of Greenwich, Connecticut as Pegosperse 200 ML. The topsheet may have a plurality of apertures to permit liquids deposited thereon to pass through to the core more quickly.

The backsheet 12 may be made of any suitable material, for example any standard backsheet material. These materials are generally flexible, liquid resistant, and liquid impervious. The general function of the backsheet is to prevent discharges absorbed by the core from escaping the sanitary napkin and soiling the clothing and bedding of the wearer.

Any conventional backsheet material may be used within the invention, such as polyolefinic films or nonwoven webs. Nonwovens webs may be advantageous because they normally provide better breathability for the articles and may be cheaper than polyolefinic films. For example, a relatively hydrophobic 23 grams per square meter (gsm) spunbonded nonwoven web of 4 denier polypropylene fibers available from BBA Neuberger (Italy) may be used.

The topsheet 11 and the backsheet 12 are preferentially peripherally joined using known techniques. The layers of the articles may also be glued to each other.

The absorbent element 13 can be made of any suitable material. Non-limiting examples of suitable liquid-absorbent materials include comminuted wood pulp which is generally referred to as airfelt; creped cellulose wadding; absorbent gelling materials including superabsorbent polymers such as hydrogel-forming polymeric gelling agents; chemically stiffened, modified, or cross-linked cellulose fibers; meltblown polymers including co-form; synthetic fibers including crimped polyester fibers; tissue including tissue wraps and tissue laminates; capillary channel fibers; absorbent foams; absorbent sponges; synthetic staple fibers; peat moss; or any equivalent material; or combinations thereof. The absorbent element preferably comprises a superabsorbent polymer (SAP), normally distributed within a matrix of cellulosic fibers, for example in order to reduce the thickness of the absorbent core.

The absorbent element may be unitary, or may be a laminate of two or more layers. For example, the core may comprise a fluid impermeable barrier layer (e.g. a PE Patch) on its backsheet-facing side to prevent fluids retained by the absorbent core from striking through the pantiliner and soiling adjacent garments. An exemplary PE patch is a 25 gsm poly film available from Britton Taco (UK) under trade name ST-012A-White.

Further generic information regarding absorbent elements can be found in prior patent publications, see for example WO0207662A1 and W09119471.

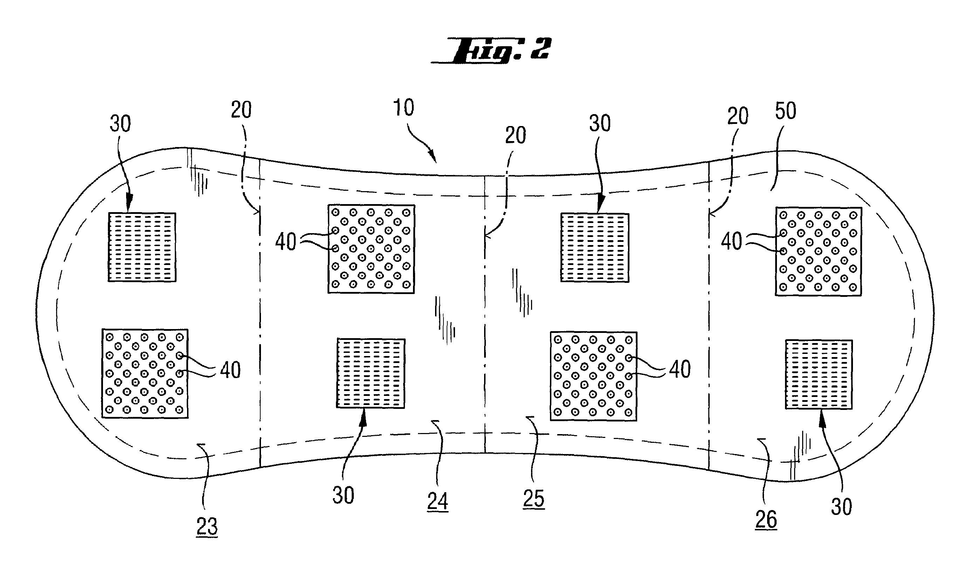

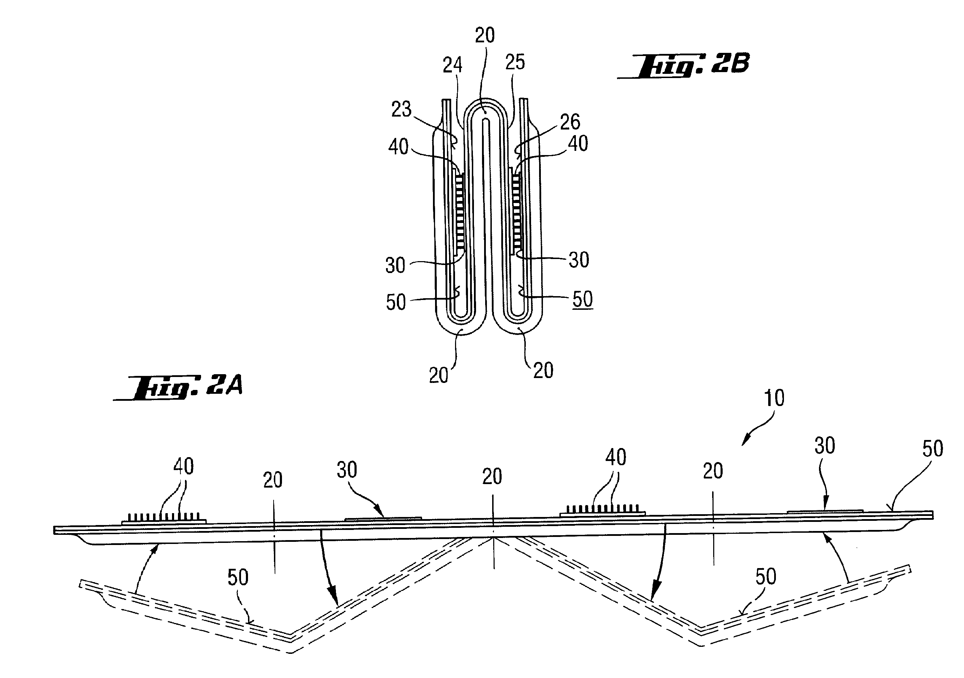

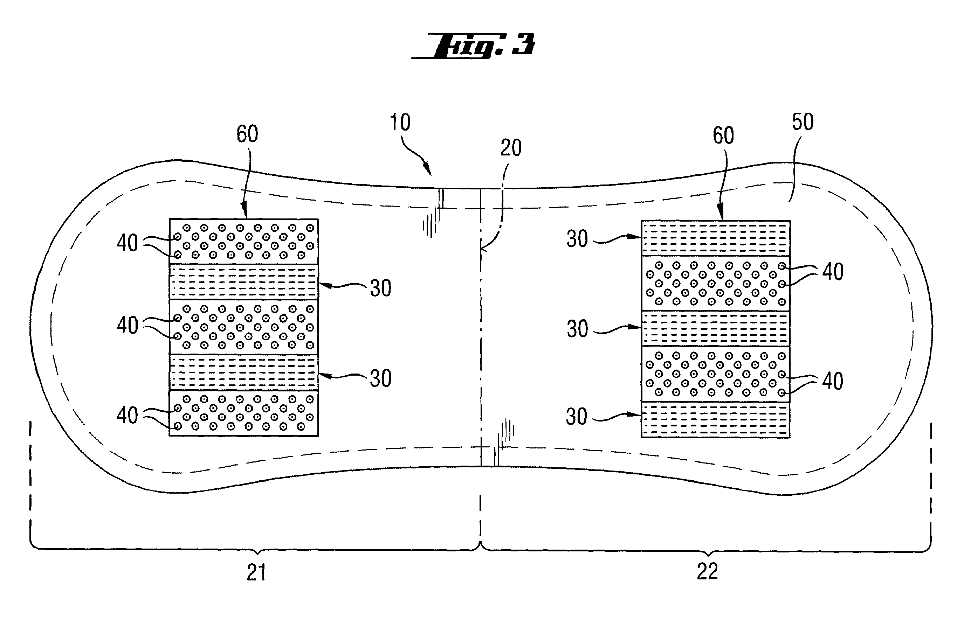

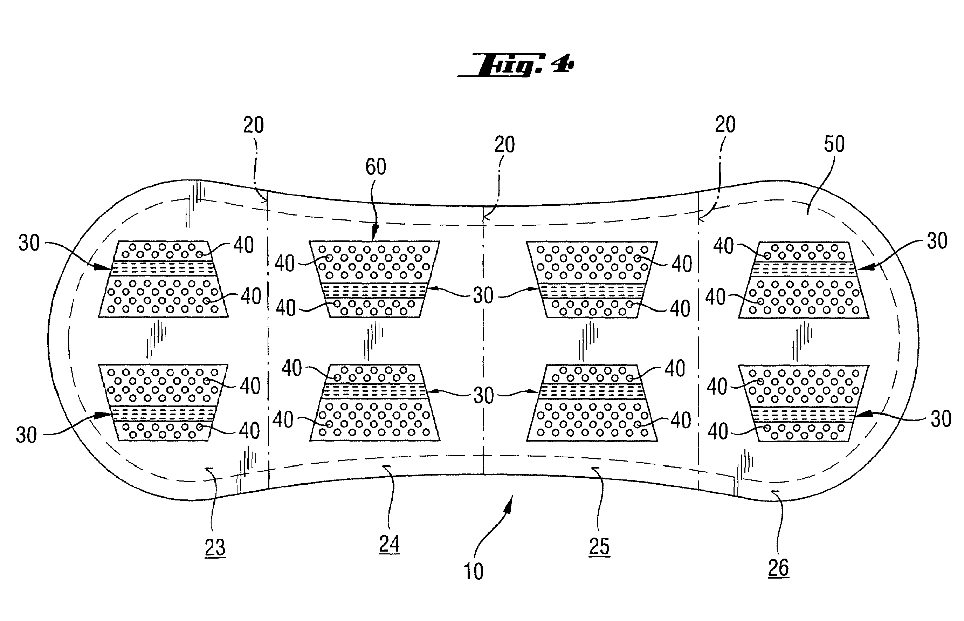

The article 10 has one or more folding lines 20 which notionally divide its garment facing surface in sections. In the exemplary embodiments depicted in Fig. 1 and 3, one folding line 20 splits the garment facing surface 50 in two portions 21 and 22. In the other exemplary embodiments depicted in Fig. 2 and 4, three folding lines 20 split the garment facing surface 50 in four portions 23, 24, 25 and 26.

The garment facing surface 50 of the article comprises an adhesive portion 30 and protrusions 40. The adhesive portion 30 and the protrusions 40 are configured so that the article can be folded along the folding lines 20 (as shown for example in Fig. 1a and 1b) so that at least a part of the adhesive portion 30 comes in contact with the protrusions 40.

When an article according to the invention is folded as described above, the part of the adhesive portion which comes in contact with the protrusions has only limited contact with the opposing part of the garment facing surface of the article because the adhesive only contacts the protrusions. The protrusions act as a spacer between the adhesive portion and the backsheet, reducing the contact of the adhesive with the garment facing surface on which it is folded. As a result the force of adhesion between the adhesive portion and the protrusions on which it is folded is reduced so that the folded article can be unfolded easily by the user and, at the same time, the adhesive is protected during storage of the folded article.

The inventors have found that such an article can be packaged in folded configuration requiring less or no release paper or film to protect the adhesive on the garment facing surface because at least part of the adhesive portion is already protected and preserved from unwanted adhesions by the contact with the protrusions. Advantageously, the adhesive portion and the protrusions are configured so that the article can be folded along the folding lines so that the adhesive portion comes in contact with the protrusions only. In this case the article can be packaged in folded configuration without requiring a release paper or film at all to protect the adhesive on its garment facing surface.

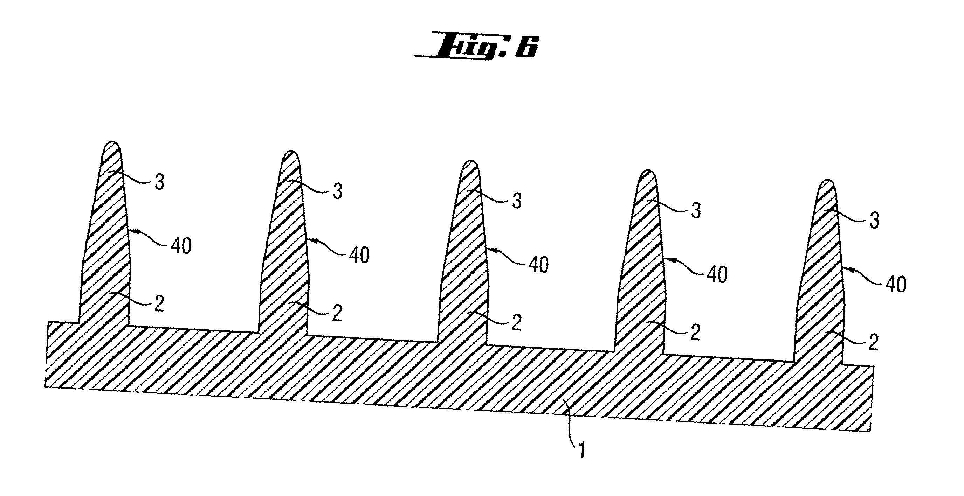

The adhesive portion of the garment facing surface of the articles may comprise a single area or may comprise a plurality of adhesive areas. In the examples shown in Fig. 1, 2, and 3 the adhesive portion comprise several discrete areas of roughly rectangular shape, in the example of Fig. 4 the shape is trapezoidal and in the example of Fig. 6 is semicircular but many other configuration can be used. The adhesive portion can represent from about 1% to about 90% or from about 2% to about 70% or from about 5% to about 50% or from about 7% to about 40% of the total garment facing surface of the article. The discrete adhesive areas can have any shape including geometric shapes (circular, oval, triangular, rectangular, square, trapezoidal, pentagonal, hexagonal etc.) or have the shape of objects, (animals, stars, letters etc.). The discrete adhesive areas may also have any size. Discrete adhesive areas can, for example, be tiny dots or long narrow stripes and can be more dense in some parts of the garment facing surface of the article and less dense in others. The different adhesive areas can have the same or different size and shape.

In the case when the adhesive portion comprises a plurality of adhesive areas, the article is configured so that it can be folded along the folding lines so that at least a part of one or more adhesive areas on the garment facing surface only comes in contact with the protrusions.

The protrusions may also act as a mechanical garment fastening system. Mechanical garment fastening systems for absorbent articles which make use of protrusions elevating from the garment facing side of the articles are known in the art. For example, systems based on hooks (e.g. Velcro™), stems or "mushrooms" have been described. Any of these can be used as protrusions in articles according to the present invention.

The protrusions acting as mechanical fastening system act in combination with the adhesive portion provide the combined benefits of a pressure sensitive adhesive fastening with those of a mechanical system (optimal resistance to lateral shifts as known to the skilled person).

The protrusions may comprise stems having a height measured along their longitudinal axis and a width corresponding to their largest dimension in a plane perpendicular to their longitudinal axis. The height of at least some, or all, of the stems may be between about 0.3 mm and about 3 mm, or between about 0.4 mm and about 2 mm and the width of at least some, or all, of the stems may be between about 0.05 mm and about 0.250 mm, or between about 0.15 mm and about 0.23 mm. The ratio of the height to the width (H/W) of some or all of the stems may be greater than about 2.

At least the tip section of the stems may be conical. The stems may be cone shaped or may comprise a first straight cylindrical lower section and at least a second conical upper section, particularly a conical tip section, the conicity of which (defined by its upper half angle) may be between 3° and 20°. Alternatively, as shown for example in the stems of Fig. 6, the stems 40 may comprise a first lower conical section 2 and at least second upper conical section 3, particularly a conical tip section, of which the conicity (defined by its upper half angle) is greater than that of the first section 2, in particular being between 3° and 20°. This at least double conicity ensures particularly efficient action of the anti-slip effect.

The stems may have a free end in the shape of a dome, particularly spherical, and particularly with a radius of curvature between 0.025 mm and 0.080 mm, or between 0.033 mm and 0.054 mm.

The axis of the stems may be inclined respective to the plane of the backsheet of the article with an angle comprised between about 70° and about 110°. Alternatively, the stems may be substantially perpendicular to this plane, with this angle being substantially equal to about 90°.

The external surface of the stems, particularly the top part of the stems, may have a rugosity measured under the Standard ISO (Ra or average deviation of rugosity, i.e. the arithmetical average of all the Y coordinates of the profile in a base length) of less than about 400 nm. In other embodiments rugosity may be less than about 300 nm, or less than about 250 nm. Alternatively at least a part of the external surface of the stems, in particular a top part of the stems, may have a statistical rugosity measured under the Standard ISO (Rms) less than about 450 nm. In other embodiments statistical rugosity may be less than about 350 nm, or less than about 275 nm.

A shape of the stem as described above and/or a rugosity or a statistical rugosity within mentioned ranges provides absorbent articles according to the present invention which are particularly delicate on fabrics and thus do not cause damage to the underwear.

In any of the embodiments described, the protrusions can be arranged in rows, circles, geometric figures or can be randomly disposed over the garment facing surface of the article. In some cases a portion of the garment facing surface of the article may comprise from 1 to about 500 protrusions per cm², or from about 10 to about 300 protrusions per cm², or from about 20 to about 200 protrusions per cm². In this context "cm²" must be intended as measured on the plane defined by the backsheet of the article with the protrusions projected on such plane. In these embodiments the protrusions can be of any of the types mentioned above.

The protrusions may be obtained by any standard methods known in the art, for example by mechanical deformation of the backsheet material or by attaching a portion of a web comprising protrusions (e.g. Velcro® or Velcro® like hooks, or mushrooms or stems) projecting out of a thin plastic base strip, onto the backsheet of the article. Such web can be typically obtained via molding of a thermoplastic material such as polyethylene or polypropylene, and can be produced in the form of a ribbon which is cut for use during the production of the articles.

For example the protrusions can be obtained by attaching on the backsheet of the article one or more portions of a web with stems, of the type described in our co-pending European patent application EP2014270.

As shown for example in the embodiments of Fig. 3 and 4, articles according to the invention can be obtained by attaching on the backsheet of the article portions of a web 60 comprising protrusions 40 wherein the same web comprises one or more adhesive portions 30. These embodiments have the advantage that the absorbent articles of the invention can be obtained by simply attaching portions of this web in the form of a ribbon comprising both adhesive portions and portions comprising protrusions in predetermined positions over the backsheet of the absorbent article.

As for example in the embodiment of Fig. 4, trapezoidal pieces of a strip of thin plastic material 60 may be applied onto the garment facing side of an article 10, each of said trapezoidal pieces comprising one or more adhesive area 30 and one or more areas comprising protrusions 40, said trapezoidal pieces 60 and the folding lines are arranged so to satisfy the requirements of the present invention. Each of said trapezoidal pieces 60 has a shape so that the length in longitudinal direction of the longest adhesive area is smaller than the length measured in the same direction of at least the longest area comprising protrusions. The trapezoidal pieces 60 can be arranged as in the figure so that the adhesive portions only come into contact with the protrusions even in case of a small manufacturing tolerance.

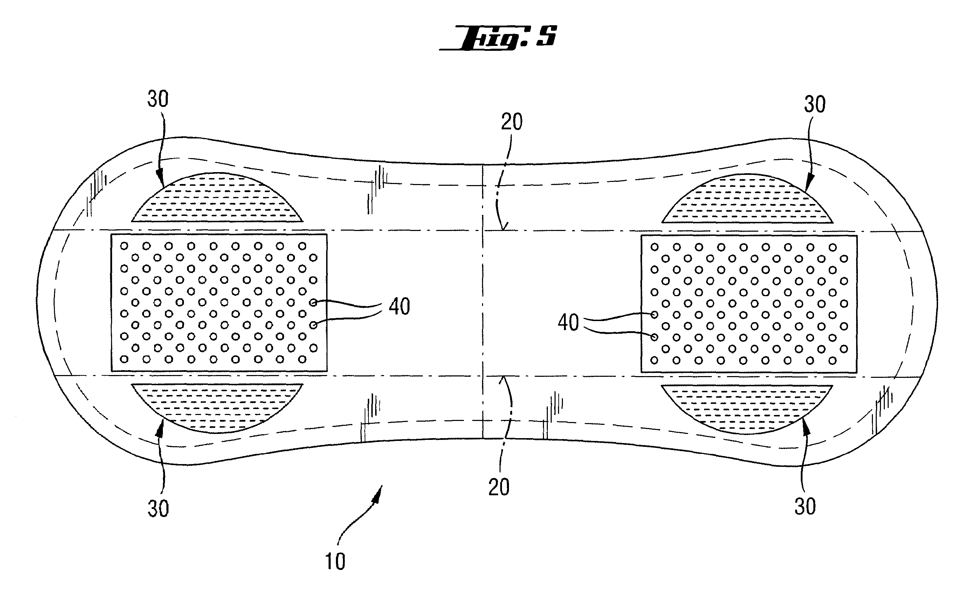

In the articles described so far, the folding lines were always directed along a transverse direction, but, provided the requirements of the invention are satisfied, they may be oriented in any direction, for example folding lines 20 may be directed along a longitudinal direction, as for example in the embodiment of Fig. 5. In some case the same article may comprise folding lines oriented in different direction e.g. orthogonal directions.

In some embodiments the articles are packaged for retail in a folded configuration, for example in a box or a bag, requiring less or no release paper or film to protect the adhesive portion. In some cases the absorbent articles in a folded configuration can be individually sealed for hygienic purposes and a simple nonwoven, paper or plastic film can be used. This offers an advantage with respect to the expensive silicone treated films which are used to act both as an adhesive release system and as a packaging wrap in individually wrapped absorbent articles such as those currently on the market as Always™ Ultra Pads.

Absorbent articles according to the invention can be easily unfolded to their flat configuration and once pressed onto the undergarment the adhesive portion will guarantee adhesion. In those embodiments where a mechanical fastener such as a web with stems is used to provide protrusions, such protrusions act as a secondary fastener penetrating the fibers of the undergarment and preventing lateral shifts of the article during use. These articles offer a further improved garment fastening performance and stay in place by combining a traditional adhesive which prevents shifting in all directions with a mechanical fastener which completely blocks lateral shifts. The advantage of using a web with stems as described in the present invention are also described in patent application EP2014270.

Absorbent articles according to the present invention can be obtained for example from any standard absorbent article like for example Always® sanitary napkins or pantyliners by replacing the pressure sensitive adhesive on the garment facing surface of the article with an adhesive portion and protrusions as described above.

In the case of absorbent articles having wings, the same construction is applicable also to them. Wings can be integral or attached extensions of the topsheet and/or the backsheet extending in transverse direction outside of the article main body and in general comprise an adhesive. When the article is in use, wings are normally folded and attached under the crotch of the underwear. Articles according to the present invention having wings may be packaged for retail, including a release paper or film specifically dedicated to the release of the adhesive on the wings. Alternatively articles with wings may comprise adhesive areas and protrusions on the wings' surface which are arranged in a way such that the article can be folded so that also the adhesive and the protrusions on the wings come in contact with each other. In this case the article can be packaged in a folded configuration without the need for a release paper or film for the adhesive on the wings.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. An absorbent article (10) for personal hygiene of the type which is positioned between the undergarment and the body of the user when in use,
said article comprising a body facing surface and a garment facing surface,
said garment facing surface comprising an adhesive portion (30), and protrusions (40) separate from said adhesive portion,
said article also comprising one or more folding lines (20);
the adhesive portion (30) and the protrusions (40) being configured so that the article (10) can be folded along the folding lines (20) so that at least a part of the adhesive portion (30) comes in contact with the protrusions (40).

2. An article (10) according to claim 1 wherein said article (10) can be folded along the folding lines (20) so that the adhesive portion (30) comes in contact with the protrusions (40) only.

3. An article (10) according to claim 1 or 2 wherein the article is a sanitary napkin, an incontinence pad or a pantiliner.

4. An article (10) according to any of the preceding claims wherein said article comprises a fluid pervious topsheet (11), a backsheet (12) and an absorbent element (13) positioned therebetween.

5. An article (10) according to any of the preceding claims comprising one, three or five folding lines (20).

6. An article (10) according to any of the preceding claims, wherein the article is folded along the folding lines (20) so that the at least a part of the adhesive portion (30) comes in contact with the protrusions (40).

7. An article (10) according to claim 6, wherein the adhesive portion (30) comes in contact with the protrusions (40) only.

8. An article (10) according to claim 6 or 7 comprising one folding line (20) and being folded in a V shaped configuration.

9. An article (10) according to claim 6 or 7 comprising three folding lines (20) and being folded in a W shaped configuration.

10. An article (10) according to any of the preceding claims wherein the adhesive portion (30) comprises a plurality of discrete adhesive areas.

11. An article according to any of the preceding claims wherein at least some of the protrusions (40) also act as mechanical fasteners for the attachment of the article onto the undergarments when the article is in use.

12. An article (10) according to claim 11 wherein the protrusions (40) comprise at least one stem having a height, measured along its longitudinal axis, and a width, corresponding to its largest dimension in a plane perpendicular to their longitudinal axis, said height being comprised between 0.3 mm and 3 mm, said width being comprised between 0.05 mm and 0.250 mm, and the ratio of the height to the width (H/W) being greater than 2.

13. An article (10) according to any preceding claim which is packaged for retail in a folded configuration, wherein said article does not comprise a release paper or film covering the adhesive portion (30).
